# EUROPEAN PATENT APPLICATION

(11) **EP 3 257 466 A1**
(43) Date of publication of application: **20.12.2017**
(21) Application number: 16174408.1
(22) Date of filing: 14.06.2016
(51) Int. Cl.: A61B 90/50, A61B 90/11, A61B 90/57

(54) **PROBE HOLDER POSITIONING DEVICE**

(71) Applicant: GFM Gesellschaft für Medizintechnik mbH, 64560 Riedstadt-Leeheim (DE)
(72) Inventor: KOBER, Bernd, 68723 Schwetzingen (DE); ALMSTEDT, Simon Fabian, 64295 Darmstadt (DE)
(74) Representative: Kramer Barske Schmidtchen Patentanwälte PartG mbB

(57) **Abstract**

A probe holder positioning device (14) for moving a probe holder (16) relative to a table (12) and locking the probe holder (16) in a desired position with respect to the table (12) is disclosed. The probe holder positioning device (14) comprises a tube (34) extending along a longitudinal axis (36) and including a longitudinal direction and a circumferential direction; a first mounting arm (18) configured to be mounted to the table (12), being attached to the tube (34) and being rotatable in the circumferential direction; a second mounting arm (20) configured to be mounted to the table (20), being attached to the tube (34) and being rotatable in the circumferential direction; and a locking device (70) attached to the tube (34) and being movable in the longitudinal direction of the tube (34) to a locking position, wherein the locking device (70) in the locking position is configured to exert a locking force (F) on the first mounting arm (18) and the second mounting arm (20) such that an angular position of the first mounting arm (18) relative to the second mounting arm (20) is locked.

## Description

### Technical Field

The present invention relates to a probe holder positioning device for moving a probe holder such as a surgical and/or diagnostic probe holder relative to a table and locking the probe holder in a desired position with respect to the table.

The present invention further relates to a medical treatment device for performing a position-based surgical and/or diagnostic medial treatment as well as to a method for positioning a probe holder.

### Background

In medical applications, medical treatments often require accurate localization of a probe holder. For example, during biopsies or interstitial brachytherapies of the prostate, a probe holder known as a stepper needs to be accurately positioned outside the patient and locked in a desired position. A probe such as an ultrasonic probe connected to the probe holder is then inserted into the rectum to support a biopsy, brachytherapie or any position-based surgical and/or diagnostic medical treatment where the biopsy or brachytherapy can be performed. Moving the probe holder and locking the probe holder can be a complex and tedious procedure for the practitioner.

The object of the present invention is therefore to provide a probe holder positioning device which facilitates moving of the probe and the probe holder and which may allow a user-friendly locking of the probe holder in a desired position.

### Summary of the Invention

The above object might be solved by a probe holder positioning device for moving a probe holder relative to a table and locking the probe holder in a desired position with respect to the table. The probe holder positioning device which may also be termed immobilizer comprises a tube, a first mounting arm and a second mounting arm rotatably attached to the tube for mounting the tube to a table such as a medical treatment table, and a locking device attached to the tube and movable in longitudinal direction of the tube to a locking position. In the locking position, the locking device is configured to exert a locking force in the longitudinal direction on the first mounting arm and the second mounting arm so that an angular position between the first mounting arm and the second mounting arm is locked. Moreover, the locking device is designed such that a single-handed use of the locking device allows locking of the probe holder in the desired position.

By providing the probe holder positioning device according to the present invention, the practitioner can move the probe holder positioning device to a desired position, hold the probe holder positioning device in the desired position with one hand and lock the probe holder positioning device with the other hand. Thus, a convenient and efficient way of performing position-based surgical and/or diagnostic medical treatments is provided.

In an exemplary embodiment of the present invention, the probe holder positioning device may also include a sliding mechanism attached to the tube and configured to move the probe holder positioning device relative to the medical treatment table. In these exemplary embodiments, the locking device is configured to exert the locking force in longitudinal direction also on the sliding mechanism so that a position of the probe holder positioning device relative to the table is locked.

In another exemplary embodiment of the present invention, the probe holder positioning device may also include a tube support device which rotatably and translationally supports the tube relative to the first and second mounting arms. In these embodiments, the locking device is configured to also exert the locking force in longitudinal direction on the tube so that a rotational and a translational movement of the tube is locked.

In another exemplary embodiment of the present invention, the tube support device of the probe holder positioning device may include one or more spring elements. The spring elements provide a spring force on the tube. The spring elements are configured such that the spring force is equal to a weight force of the tube and a weight force of the probe holder including the probe. By providing the tube support device with spring elements, handling of the probe holder positioning device, which can easily weigh several kilos, is facilitated. In addition, due to the weight balancing, positioning of the probe holder and the probe becomes more accurate.

The present invention further relates to a medical treatment device for performing a position-based surgical and/or diagnostic medical treatment. The medical treatment device comprises a table such as a medical treatment table for a patient, and a probe holder positioning device as exemplarily disclosed herein and connected to the table via the first mounting arm and the second mounting arm.

The present invention further relates to a method for positioning a probe holder with a probe connected to the probe holder using the probe holder positioning device as exemplarily disclosed herein. The method comprises connecting the probe holder positioning device to a table such as a medical treatment table via the first mounting arm and the second mounting arm, connecting the probe holder to the probe holder positioning device, connecting a diagnostic or therapeutical probe to the probe holder; providing a desired position of the probe holder positioning device relative to the table, moving the probe holder positioning device to the desired position, and moving the locking device to the locking position to lock the probe holder positioning device in the desired position.

The present invention further relates to a probe holder positioning device comprising a tube extending along a longitudinal axis and being rotatable about and movable along the longitudinal axis, and a tube support device configured to rotatably and translationally support the tube, wherein the tube support device includes at least one spring element for providing a spring force on the tube. The at least one spring element is configured such that the spring force is directed towards the tube and is about equal to a cumulative weight force generated by the tube and a probe holder connected to the tube and a probe connected to the probe holder. Preferably, the spring element is a constant force spring element.

Other features and aspects of the invention will be apparent from the following description and the accompanying drawings.

### Brief Description of the Drawings

The accompanying drawings, which are incorporated herein and constitute a part of the specification, illustrate exemplary embodiments of the disclosure and, together with the description, serve to explain the principles of the disclosure. In the drawings:
Fig. 1 shows an exemplary medical treatment device including an exemplary probe holder positioning device attached to a table;
Fig. 2 shows the exemplary probe holder positioning device of Fig. 1 in more detail;
Fig. 3 shows a cross-sectional view of an exemplary locking device of the exemplary probe holder positioning device of Fig. 2; and
Fig. 4 shows a cross sectional view of another exemplary locking device.

### Detailed Description

The following is a detailed description of exemplary embodiments of the present invention. The exemplary embodiments described herein are intended to teach the principles of the present invention, enabling those of ordinary skill in the art to implement and use the present invention in many different environments and for many different applications. Therefore, the exemplary embodiments are not intended to be, and should not be considered as a limiting description of the scope of protection. Rather, the scope of protection shall be defined by the appended claims.

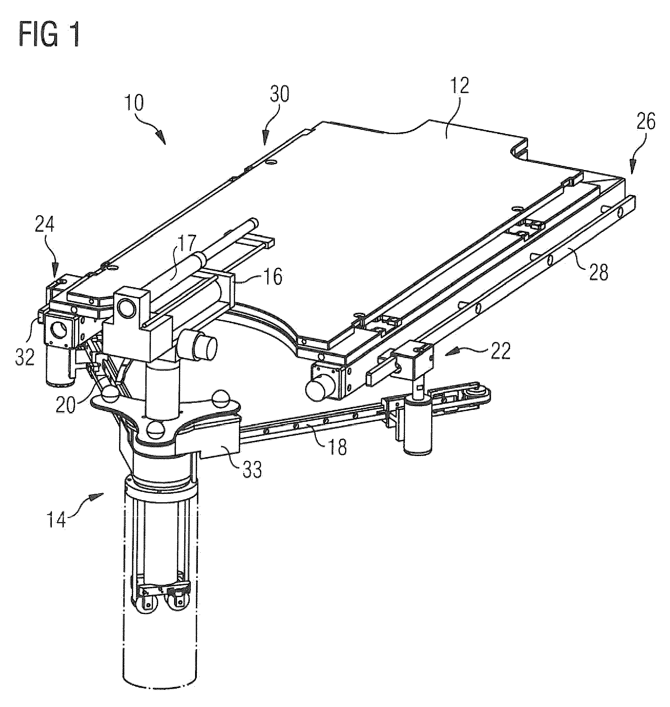

Fig. 1 shows an exemplary medical treatment device 10. Medical treatment device 10 includes a table 12 such as a medical treatment table. A patient can be placed on table 12 so that a medical treatment, for example, a biopsy, a brachytherapy or the like can be performed on the patient. Medical treatment device 10 further includes a probe holder positioning device 14 and a probe holder 16 connected to probe holder positioning device 14. Probe holder 16 may include any kind of probes 17 used for diagnostic, surgical and/or therapeutical treatments, for example a brachytherapy probe for performing a brachytherapy, a cryotherapy probe, a fiducial marker placement probe, a mapping biopsy probe, a saturation biopsy probe, a targeted biopsy probe, a volume studies probe, a template guided procedure probe or any other probe which requires a positioning system such as probe holder positioning device 14.

Probe holder positioning device 14 is movable relative to table 12 so that probe holder 16 can be positioned at a desired position relative to table 12. For this, probe holder positioning device 14 includes a first mounting arm 18 movably connected to table 12 and a second mounting arm 20 movably connected to table 12. First mounting arm 18 includes a first bearing element 22 and second mounting arm 20 includes a second bearing element 24. First bearing element 22 is configured to slide along a first table side 26. First bearing element 22 may be a linear bearing connected to first table side 26 via a first rail 28. Second bearing element 24 is configured to slide along a second table side 30. Second bearing element 24 may be a linear bearing connected to a second table side 30 via a second rail 32. As shown in Fig. 1, second table side 30 is opposite first table side 26. In other embodiments, second table side 30 may not be opposite first table side 26. Second table side 30 may even be on the same side as first table side 28.

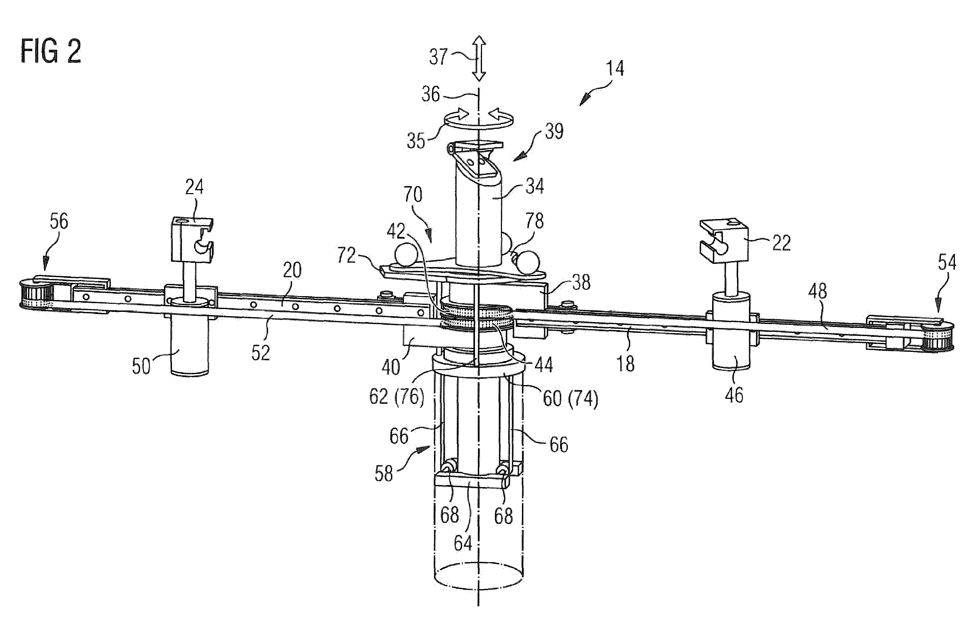

Referring now to Fig. 2, an exemplary probe holder positioning device 14 is shown in more detail. For clarity, probe holder 16 is not shown in Fig. 2.

Probe holder positioning device 14 includes a tube 34 extending along a longitudinal axis 36. Tube 34 is rotatable in circumferential direction 35 and movable in longitudinal direction 37 with respect to longitudinal axis 36. Tube 34 further includes a probe holder receiving portion 39 for receiving probe holder 16. Probe holder 16 can be fixedly or pivotally be connected to tube 34. A fine-tuning device such as an incremental positioning encoder may be interconnected between probe holder 16 and tube 34.

As can be seen in Fig. 2, first mounting arm 18 is connected to tube 34 via a first bracket 38 and second mounting arm 20 is connected to tube 34 via a second bracket 40. First bracket 38 and second bracket 40 are lugs extending in circumferential direction around tube 34. In the specific embodiment shown in Fig. 2, first bracket 38 is positioned on top of second bracket 40 when viewed from bottom to top of longitudinal axis 36.

As can be further seen in Fig. 2, a first carrier device 42 and a second carrier device 44 are attached to tube 34 inbetween first bracket 38 and second bracket 40. As exemplarily shown in Fig. 2, first carrier device 42 may be a first synchronous pulley 42 and second carrier device 44 may be a second synchronous pulley 44. In other embodiments, however, first carrier device 42 and second carrier device 44 may be first and second carrier plates configured to carry first and second belts 48, 52.

First synchronous pulley 42 and second synchronous pulley 44 are rotatable in the circumferential direction about longitudinal axis 36. First bearing element 22 includes a first connection element 46 with a substantially cylindrical shape. First connection element 46 is connected to first synchronous pulley 42 via a first belt 48. Second bearing element 24 includes a second connection element 50 with a substantially cylindrical shape. Second connection element 50 is connected to second synchronous pulley 44 via a second belt 52. First and second belts 48, 52 are tensioned by first and second belt tensioners 54, 56 which are provided at distal ends of first and second mounting arms 18, 20.

The movement of first synchronous pulley 42, second synchronous pulley 44, first bearing element 22, second bearing element 24, first belt 48 and second belt 52 results in a sliding movement of probe holder positioning device 14 relative to table 12. The first synchronous pulley 42, the second synchronous pulley 44, the first bearing element 22, the second bearing element 24, the first belt 48 and the second belt 52 may therefore constitute a sliding mechanism of probe holder positioning device 14.

Moreover, because first synchronous pulley 42 is connected to first bearing element 22 via first belt 48 and second synchronous pulley 44 is connected to second bearing element 24 via second belt 52, a movement of probe holder positioning device 14 relative to table 12 results in a rotation of first synchronous pulley 42 and second synchronous pulley 44 relative to tube 34.

Likewise, because first mounting arm 18 is connected to table 12 via first bearing element 22 and second mounting arm 20 is connected to table 12 via second bearing element 24, a movement of probe holder positioning device 14 relative to table 12 results in a rotation of first bracket 38 and second bracket 40 relative to each other. Thus, depending on a position of probe holder positioning device 14 relative to table 12, first mounting arm 18 and second mounting arm 20 may form a different angle between them, i.e. may have a different angular position to each other.

As mentioned, tube 34 is rotatable in circumferential direction 35 and movable in longitudinal direction 37 with respect to longitudinal axis 36. Thus, in addition to the above mentioned movements of first and second mounting arms 18, 20 and first and second synchronous pulleys 42, 44, tube 34 can itself rotate about and translate along longitudinal axis 36.

For rotating and translating tube 34, probe holder positioning device 14 includes a tube support device 58. Tube support device 58 is fixedly connected to first mounting arm 18 or second mounting arm 20 and is arranged coaxially with tube 34. Tube support device 58 includes a flange 60 which, in the specific embodiment shown in Fig. 2, is connected via rods 62 and a housing (item 33 in Fig. 1) to first mounting arm 18. Tube support device 58 further includes a support plate 64. Support plate 64 is configured such that a bottom end of tube 34 rests on support plate 64. Tube support device 58 further includes two spring elements 66. As exemplarily shown, spring elements 66 may be constant force spring elements. In other embodiments, however, spring elements 66 may be, for example, pneumatic springs or other types of springs. One end of each of the two constant force spring elements 66 is connected to flange 60. The other, wound-in end of each of the two constant force spring elements 66 is supported on support plate 64 via a rotatable spool element 68. Thus, when tube 34 is translated downwards, constant force spring elements 66 extend and exert a spring force on tube 34 to counterbalance a weight force of tube 34, probe holder 16 and probe 17 connected to probe holder 16. In other words, spring elements 66 are configured such that the resulting spring force exerted on tube 34 is equal to the cumulative weight force of tube 34, probe holder 16 and probe 17 connected to probe holder 16.

When spring elements 66 are constant force spring elements 66, the spring force applied to tube 34 is independent of an extension of spring elements 66. Thus, always the total weight of tube 34, probe holder 16 and probe 17 is counterbalanced by spring elements 66 independent of the spring extension. By using constant force spring elements 66, a zero-gravity feel during moving of probe holder positioning device 14 is provided which allows for a convenient and accurate handling of probe holder positioning device 14. In addition, due to the weight compensation, probe holder positioning device 14 can be released in any desired position without affecting a translational movement of probe holder positioning device 14.

Probe holder positioning device 14 further includes a locking device 70. Locking device 70 is configured to lock a desired position of probe holder positioning device 14 so that a position of probe holder positioning device 14 is fixed. Locking device 70 includes a first support member 72, a second support member 74 and a locking element 78. First support member 72 is supported on first mounting arm 18 via housing 33 (see Fig. 1). Second support member 74 is fixedly connected to first support member 72 via rods 76. Locking element 78 is movably positioned inside first support member 72 and configured to lock a desired position of probe holder positioning device 14 as will be explained below. As becomes obvious from Fig. 2, second support member 74 can integrally be formed in flange 60. In other embodiments this may, however, not be the case.

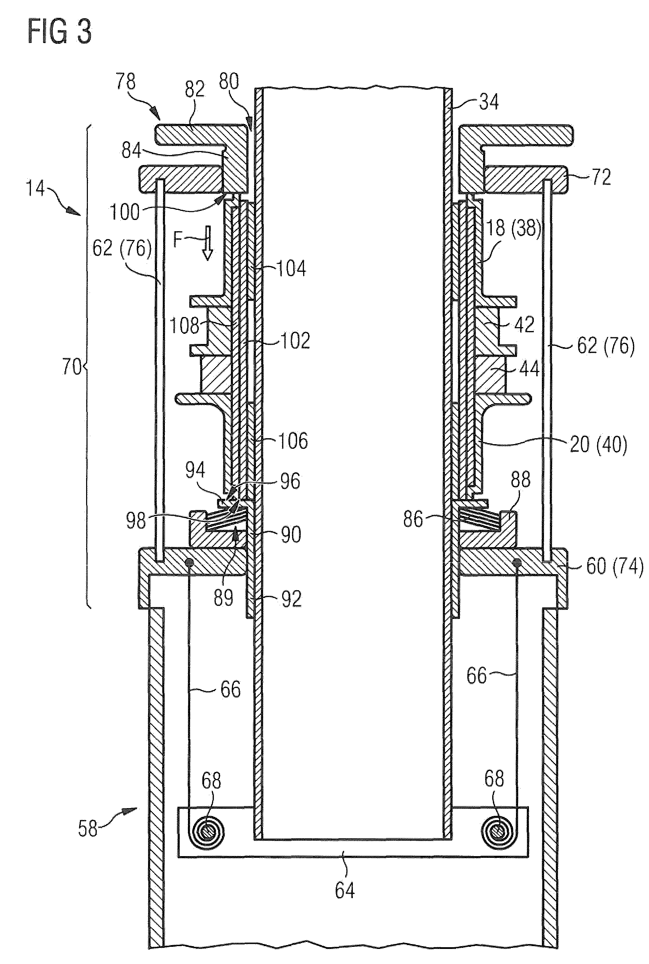

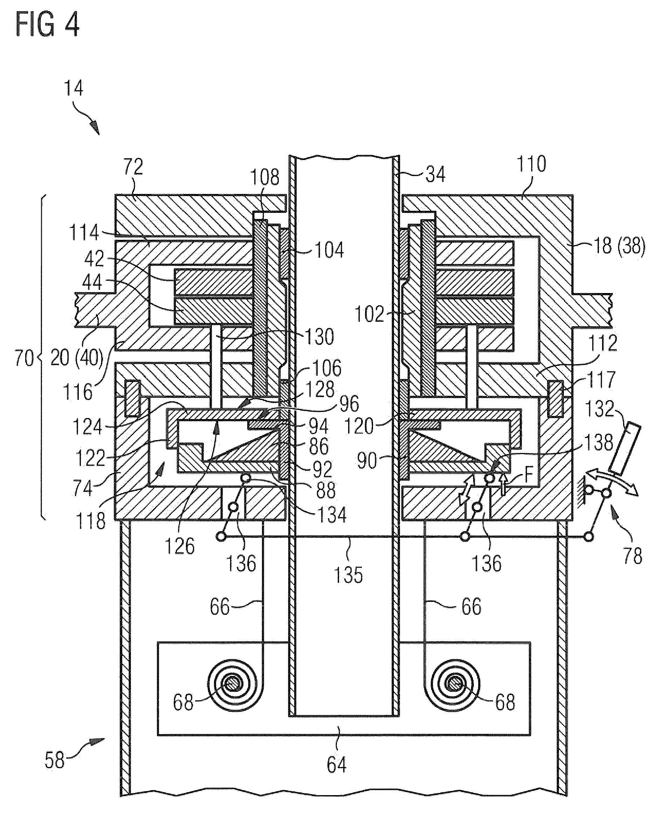

Referring now to Fig. 3, locking of probe holder positioning device 14 is explained in more detail. Fig. 3 is a cross-sectional view of probe holder positioning device 14 of Fig. 2 and depicts an exemplary embodiment of locking device 70. Another exemplary embodiment of locking device 70 will be explained in connection with Fig. 4.

First support member 72 includes a central bore 80. Locking element 78 is movably positioned in first support member 72. Locking element 78 includes an annular disc 82 and a cylindrical protrusion 84 extending from annular disc 82. Cylindrical protrusion 84 is threaded on the outside. Central bore 80 is configured to threadingly receive cylindrical protrusion 84. Thus, by turning locking element 78 around longitudinal axis 36, cylindrical protrusion 84 moves inside central bore 80 towards second support member 74.

As can be further seen in Fig. 3, first bracket 38 is arranged below first support member 72. First synchronous pulley 42 is arranged below first bracket 38. Second synchronous pulley 44 is arranged below first synchronous pulley 42. And second bracket 40 is arranged below second synchronous pulley 44.

Probe holder positioning device 14 further includes a plurality of star discs 86 and a star disc support element 88. Star disc 86 is a flat conically shaped ring made of spring steel. By applying an axial actuating force on a top face of the conical ring, an elastic change in a conical angel between portions of the ring occurs so that an inner diameter of the ring reduces. Star discs 86 extend circumferentially around tube 34 and include an inner diameter slightly larger than an outer diameter of tube 34. Star disc support element 88 rests on a top face of second support member 74 and includes an annular recess 89 configured to receive star discs 86. Annular recess 89 is configured to axially support star discs 86 and radially support an outer circumference of star discs 86.

Probe holder positioning device 14 further includes a star disc bearing bushing 90 arranged in radial direction between tube 34 and star disc support element 88. Star disc bearing bushing 90 has a substantially L-shaped cross-sectional shape including a cylindrical leg 92 and a radial shoulder 94. Cylindrical leg 92 extends in longitudinal direction of tube 34 between tube 34 and star disc support element 88 and flange 60. Cylindrical leg 92 is sized such that an inner circumference of cylindrical leg 92 abuts an outer circumference of tube 34. Moreover, cylindrical leg 92 is sized such that an outer circumference of cylindrical leg 92 abuts an inner circumference of star discs 86, an inner circumference of star disc support element 88 and an inner circumference of flange 60. Radial shoulder 94 extends radially outward from cylindrical leg 92. A radial distance of radial shoulder 94 is sized such that radial shoulder 94 at least partially covers star discs 86 from on top. An axial distance of radial shoulder 94 is sized such that a top face 96 of radial shoulder 94 abuts a bottom face 98 of second bracket 40.

Thus, when locking element 78 is turned around longitudinal axis 36 and moved through central bore 80 towards second support member 74, a force F is exerted in longitudinal direction from a force transmission face 100 of cylindrical protrusion 84 to a top face of first bracket 38. Force F is then transmitted through friction from a bottom face of first bracket 38 to a top face of first synchronous pulley 42, from a bottom face of first synchronous pulley 42 to a top face of second synchronous pulley 44, from a bottom face of second synchronous pulley 44 to a top face of second bracket 40, from bottom face 98 of second bracket 40 to top face 96 of radial shoulder 94 and from a bottom face of radial shoulder 94 to a top face of star discs 86. Upon exerting force F on star discs 86, an inner diameter of star discs 86 reduces so that star disc bearing bushing 90 exerts a radial force on an outer circumferential face of tube 34.

As a result of the force exertion, first and second brackets 38, 40 are locked in their rotational movement, first and second synchronous pulleys 42, 44 are locked in their rotational movement and tube 34 is locked in its rotational and translational movement. Hence, upon the force exertion, an angular position between first and second mounting arms 18, 20 is locked, a rotation of first and second synchronous pulleys 42, 44 (and thus a position of probe holder positioning device 14 relative to table 12) is locked and a rotation and a translation of tube 34 relative to tube support device 58 is locked. In other words, when locking element 78 is moved towards second support member 74, all rotational and translational degrees of freedom of probe holder positioning device 14 are locked. Or in other words, by turning locking element 78, locking element 78 is moved from a release position where a rotational and translational movement of probe holder positioning device 14 is not locked to a locking position where a rotational and translational movement of probe holder positioning device 14 is locked.

Locking device 70 is further designed such that locking element 78 can be actuated single-handedly. Thus, an operator of probe holder positioning device 14 can move probe holder 16 to a desired position, hold probe holder 16 with one hand in the desired position and lock probe holder 16 at the desired position with the other hand.

As mentioned, first and second synchronous pulleys 42, 44 are connected to first and second bearing elements 22, 24 via first and second belts 48, 52. Hence, upon sliding probe holder positioning device 14 relative to table 12, a radial force is exerted on first and second synchronous pulleys 42, 44 by first and second belts 48, 52. The radial force exertion may cause uneven rotation of first and second synchronous pulleys 42, 44 and may further cause a jerking or unsmooth rotation and translation of tube 34. To prevent such an unsmooth movement of tube 34, probe holder positioning device 14 further includes a tube sleeve 102. Tube sleeve 102 is arranged in circumferential direction between tube 34 and first and second brackets 38, 40 as well as between tube 34 and first and second synchronous pulleys 42, 44. A bracket bushing 108 is arranged in circumferential direction between tube sleeve 102 and first and second brackets 38, 40. In addition, a first tube bushing 104 and a second tube bushing 106 is arranged in circumferential direction between tube 34 and tube sleeve 102. Thus, any radial force acting on first and second synchronous pulleys 42, 44, is transmitted from first and second synchronous pulleys 42, 44 via bracket bushing 108 and tube sleeve 102 to first and second brackets 38, 40. By using tube sleeve 102, a smooth rotation and a smooth translation of tube 34 during sliding of probe holder positioning device 14 relative to table 12 is ensured.

Referring now to Fig. 4, a second embodiment of locking device 70 is shown. Compared to the locking device of Fig. 3, locking element 78 is a lever device.

Explaining locking device 70 in more detail, first bracket 38 includes a substantially U-shaped cross-sectional shape with a first upper leg 110 and a first lower leg 112. First support member 72 is integrally formed in first upper leg 110. Second bracket 40 includes a substantially U-shaped cross-sectional shape with a second upper leg 114 and a second lower leg 116. Second bracket 40 is arranged inside first bracket 38, i.e. second bracket 40 is arranged between first upper leg 110 and first lower leg 112. First synchronous pulley 42 and second synchronous pulley 44 are arranged inside second bracket 40, i.e. first synchronous pulley 42 and second synchronous pulley 44 are arranged between second upper leg 114 and second lower leg 116. Second support member 74 is connected to first lower leg 112 via connection elements 117 such as screws. Second support member 74 may also integrally be formed in second bracket 40. Second support member 74 includes an annular recess 118. Annular recess 118 is configured to receive star discs 86, star disc bearing bushing 90 and star disc support element 88.

Probe holder positioning device 14 further includes a collar bushing 120 arranged on top of star disc bearing bushing 90, star discs 86 and star disc support element 88. Collar bushing 120 includes a cylindrical leg 122 and a radial collar 124. Radial collar 124 extends in radial direction from cylindrical leg 122 towards tube 34. A radial distance of radial collar 124 is such that radial collar 124 covers at least partially radial shoulder 94. An inner diameter of cylindrical leg 122 is such that an inner circumference of cylindrical leg 122 abuts an outer circumference of star disc support element 88. A height of cylindrical leg 122 is such that a bottom face 126 of collar bushing 120 abuts top face 96 of radial shoulder 94. Radial collar 124 further includes a top face 128 opposite bottom face 126. A pin 130 is arranged on top face 128 and extends in longitudinal direction of tube 34 through a through-hole in first lower leg 112. Pin 130 is further slidably supported in second lower leg 116, extends through second lower leg 116 and abuts a bottom face of second synchronous pulley 44.

Lever device 78 includes a lever member 132 and two actuation members 134. Lever member 132 is arranged in the vicinity of tube 34, i.e. radial outward of first and second brackets 38, 40. Actuation members 134 are connected to lever member 132 via rods 135. Actuation members 134 extend through bores 136 of second support member 74 and are movable in longitudinal direction of tube 34.

Thus, when lever member 132 is pivoted from a release position to a locking position, rods 135 translate the pivotal movement into a translational movement of actuation members 134. Actuation members 134 then move through bores 136 and exert a force F from a force transmission face 138 to a bottom face of star disc support element 88. Force F is then transmitted from a top face of star disc support element 88 to a bottom face of star discs 86, from a top face of star discs 86 to a bottom face of radial shoulder 94, from top face 96 of radial shoulder 94 to bottom face 126 of radial collar 124, from top face 128 of radial collar 124 to pins 130 and from pins 130 to a bottom face of second synchronous pulley 44. Force F is then further transmitted through friction from a top face of second synchronous pulley 44 2 to a bottom face of first synchronous pulley 42, from a top face of first synchronous pulley 42 to a bottom face of second upper leg 114, and from a top face of second upper leg 114 to a bottom face of first upper leg 110.

As a result of the force exertion, first and second brackets 38, 40 are locked in their rotational movement, first and second synchronous pulleys 42, 44 are locked in their rotational movement and tube 34 is locked in its rotational and translational movement. Hence, upon the force exertion, an angular position between first and second mounting arms 18, 20 is locked, a rotation of first and second synchronous pulleys 42, 44 (and thus a position of probe holder positioning device 14 relative to table 12) is locked and a rotation and a translation of tube 34 relative to tube support device 58 is locked. In other words, when lever member 132 is pivoted from the release position into the locking position, all rotational and translational degrees of freedom of probe holder positioning device 14 are locked.

Again, locking device 70 is designed such that lever member 132 can be actuated single-handedly. Thus, an operator of probe holder positioning device 14 can move probe holder 16 to a desired position, hold prober holder 16 at the desired position with one hand and lock probe holder 16 at the desired position with the other hand. Moreover, due to the defined pivoting of lever member 132, a predetermined locking force F is provided.

As illustrated in Fig. 4, probe holder positioning device 14 includes tube sleeve 102, first tube bushing 104, second tube bushing 106 and bracket bushing 108 to transmit radial forces acting on first and second synchronous pulleys 42, 44 to first and second brackets 38, 40.

By comparing the embodiments of the locking device 70 shown in Figs. 3 and 4, it becomes obvious that first bracket 38 of first mounting arm 18, second bracket 40 of second mounting arm 20, first synchronous pulley 42, second synchronous pulley 44 and star disc 86 are all arranged between first and second support members 72,74. In other words, the rotational and translational components attached to tube 34 are all arranged between first support member 72 and second support member 74. Moreover, when locking element 78 is moved from the release position to the locking position, a distance between force transmission face 100 and second support member 74 or a distance between force transmission face 138 and first support member 72 is reduced. As a result, locking element 78 exerts a compressive force F on the rotational and translational components arranged between first and second support member 72, 74 so that a frictional force locks the rotational and translational components in its respective positions.

Terms such as "about", "around", "approximately", or "substantially" as used herein when referring to a measurable value such as a parameter, an amount, a temporal duration, and the like, is meant to encompass variations of ±10% or less, preferably ±5% or less, more preferably ±1% or less, and still more preferably ±0.1% or less of and from the specified value, insofar as such variations are appropriate to perform in the disclosed invention. It is to be understood that the value to which the modifier "about" refers is itself also specifically, and preferably, disclosed. The recitation of numerical ranges by endpoints includes all numbers and fractions subsumed within the respective ranges, as well as the recited endpoints.

Terms "above", "on top", or "below" as used herein, are used in reference to longitudinal axis 36.

### Industrial Applicability

The probe holder positioning device according to the present invention is applicable to any position-based medical treatment such as interstitial brachytherapy treatments, gynecological applications or interventions and/or surgical interventions.

In the following, operation and control of probe holder positioning device 14 is explained in connection with Figs. 1 to 4. For illustration purposes, the control procedures described herein are disclosed with reference to structural elements disclosed in Figs. 1 to 4. However, the skilled person will appreciate that the respective steps of the control procedure can be performed on other embodiments as well.

In a first step, probe holder positioning device 14 is connected to table 12. For this, first mounting arm 18 is connected to first table side 26 via first bearing element 22 and second mounting arm 20 is connected to second table side 30 via second bearing element 24.

In a next step, probe holder 16 is connected to probe holder receiving portion 39 of probe holder positioning device 14. Of course, first and second steps may be performed in alternate sequence.

In a next step, probe 17 used for diagnostic, surgical and/or therapeutical treatments is connected to probe holder 16. Of course, probe 17 may already be connected to probe holder 16 when probe holder 16 is connected to probe holder positioning device 14.

In a next step, a desired position of probe holder positioning device 14 relative to table 12 is provided. The desired position may be predetermined or may be set by the practitioner.

In a next step, probe holder positioning device 14 is moved to the desired position. During movement of probe holder positioning device 14, first and second bearing elements 22, 24 may slide along first and second rails 28, 32. During this sliding movement, first and second synchronous pulleys 42, 44 rotate relative to tube 34 so that first mounting arm 18 adopts an angular position relative to second mounting arm 20. In addition, during movement of probe holder positioning device 14, tube 34 may be rotated about longitudinal axis 36 and/or may be translated along longitudinal axis 36. As tube 34 is supported by tube support device 58, a full weight compensation of tube 34 and probe holder 16 including probe 17 is ensured providing the practitioner with a zero-gravity feel. In addition, when probe holder positioning device 14 is positioned at the desired position, a practitioner can release probe holder positioning device 14 without affecting the desired position.

In a next step, the practitioner then either rotates annular disc 82 about longitudinal axis 36 or pivots lever member 132 to lock the desired position of probe holder positioning device 14. During rotation of annular disc 82 and during pivoting of lever member 132, a compressive force F is exerted on first bracket 38, on second bracket 40, on first synchronous pulley 42, on second synchronous pulley 44 and on star discs 86. By exerting the compressive force F, an angular position between first mounting arm 18 and second mounting arm 20, a rotation of first and second synchronous pulleys 42, 44 and a rotation and a translation of tube 34 is locked so that probe holder positioning device 14 is locked in the desired position.

By providing the probe holder positioning device according to the present invention, a convenient and efficient way of moving and locking a probe holder is provided. In particular, the practitioner can move the probe holder to a desired position, hold probe holder at the desired position with one hand and lock the probe holder with the other hand. Moreover, due to the full weight compensation, a zero-gravity feel during moving and handling of the probe holder positioning device and the probe holder including the probe is provided.

Although the preferred embodiments of this invention have been described herein, improvements and modifications may be incorporated without departing from the scope of the following claims.

## Claims

1. A probe holder positioning device (14) for moving a probe holder (16) relative to a table (12) and locking the probe holder (16) in a desired position with respect to the table (12), the probe holder positioning device (14) comprising:
a tube (34) extending along a longitudinal axis (36) and providing a longitudinal direction and a circumferential direction;
a first mounting arm (18) configured to be mounted to the table (12), the first mounting arm (18) being attached to the tube (34) and being rotatable in the circumferential direction;
a second mounting arm (20) configured to be mounted to the table (12), the second mounting arm (20) being attached to the tube (34) and being rotatable in the circumferential direction; and
a locking device (70) attached to the tube (34) and being movable in the longitudinal direction to a locking position, the locking device (70) being configured to exert a locking force (F) on the first mounting arm (18) and the second mounting arm (20) such that an angular position of the first mounting arm (18) relative to the second mounting arm (20) is locked.

2. The probe holder positioning device (14) of claim 1,
wherein the tube (34) is rotatable about and movable along the longitudinal axis (36).

3. The probe holder positioning device (14) of claim 2, further comprising:
a tube support device (58) configured to rotatably and translationally support the tube (34) relative to the first mounting arm (18) and the second mounting arm (20), wherein the locking device (70) is configured to exert the locking force (F) also on the tube (34) such that a rotational and a translational movement of the tube (34) is locked.

4. The probe holder positioning device (14) of claims 2 or 3, wherein the tube support device (58) is arranged coaxially with the tube (34) and the probe holder positioning device (14) further comprises:
at least one star disc (86) extending circumferentially around the tube (34) and being supported axially and radially on the tube support device (58), wherein the locking device (70) is configured to exert the locking force (F) also on the at least one star disc (86).

5. The probe holder positioning device (14) of any one of the preceding claims, further comprising:
a sliding mechanism attached to the tube (34) adjacent the first mounting arm (18) and the second mounting arm (20) and being configured to move the probe holder positioning device (14) relative to the table (12), wherein the locking device (70) is configured to exert the locking force (F) also on the sliding mechanism such that a position of the probe holder positioning device (14) relative to the table (12) is locked.

6. The probe holder positioning device (14) of claim 5, wherein the sliding mechanism includes:
a first bearing element (22) connected to the first mounting arm (18) and configured to translationally move the first mounting arm (18) along a first table side (26);
a second bearing element (24) connected to the second mounting arm (20) and configured to translationally move the second mounting arm (20) along a second table side (30);
a first carrier device (42) rotatably attached to the tube (34) and connected to the first bearing element (22) via a first belt (48); and
a second carrier device (44) rotatably attached to the tube (34) and connected to the second bearing element (24) via a second belt (52), wherein the locking device (70) is configured to exert the locking force (F) on the first carrier device (42) and the second carrier device (44) such that a rotation of the first carrier device (42) and a rotation of the second carrier device (44) is locked.

7. The probe holder positioning device (14) of claim 6, wherein the first mounting arm (18) is connected to the tube (34) via a first bracket (38), the second mounting arm (20) is connected to the tube (34) via a second bracket (40), the first bracket (38) is arranged on top of the first carrier device (42), the first carrier device (42) is arranged on top of the second carrier device (44), the second carrier device (44) is arranged on top of the second bracket (40) and the locking device (70) is configured to transmit the locking force (F) from the first bracket (38) to the first carrier device (42), from the first carrier device (42) to the second carrier device (44) and from the second carrier device (44) to the second bracket (40).

8. The probe holder positioning device (14) of claim 6, wherein the first mounting arm (18) is connected to the tube (34) via a first bracket (38) having a substantially U-shaped cross-sectional shape, the second mounting arm (20) is connected to the tube (34) via a second bracket (40) having a substantially U-shaped cross-sectional shape, the second bracket (40) is arranged inside the first bracket (38), the first carrier device (42) is arranged on top of the second carrier device (44), the first carrier device (42) and the second carrier device (44) are arranged inside the second bracket (40) and the locking device (70) is configured to transmit the locking force (F) from the second carrier device (44) to the first carrier device (42), from the first carrier device (42) to the second mounting arm (20) and from the second mounting arm (20) to the first mounting arm (18).

9. The probe holder positioning device (14) of any one of the preceding claims, wherein the locking device (70) further includes:
a first support member (72) fixedly connected to the first mounting arm (18) or the second mounting arm (20);
a second support member (74) fixedly connected to the first support member (72); and
a locking element (78) movable in longitudinal direction within one of the first support member (72) and the second support member (74) to reduce a distance between the locking element (78) the other of the first support member (72) and the second support member (74).

10. The probe holder positioning device (14) of claim 9, wherein the locking element (78) is rotatable or pivotable.

11. The probe holder positioning device (14) of claim 10, wherein the locking element (78) includes a cylindrical protrusion (84) threadingly received in the first support member (72)
or
wherein the locking element (78) includes a lever member (132) and an actuation member (134) connected to the lever member (132), the lever member (132) being pivotally arranged outside of the tube (34) and the actuation member (134) being movably arranged inside the second support member (74).

12. The probe holder positioning device (14) of claim 2,
wherein the tube support device (58) includes a spring element (66) for providing a spring force on the tube (34), the spring element (66) being configured such that the spring force is about equal to a cumulative weight force of the tube (34), the probe holder (16) connected to the tube (34) and a probe (17) connected to the probe holder (16).

13. The probe holder positioning device (14) of any one of the preceding claims, further comprising:
a tube sleeve (102) arranged in circumferential direction between the tube (34) and the first mounting arm (18) and the second mounting arm (20);
and/or
a probe holder receiving portion (39) arranged on a top face of the tube (34) and configured to mount the probe holder (16) to the tube (34).

14. A medical treatment device (10) for performing a position-based surgical and/or diagnostic medial treatment, the medical treatment device comprising:
a table (12) configure to support a patient; and
a probe holder positioning device (14) according to any one of the preceding claims, wherein the probe holder positioning device (14) is connected to the table (12) via the first mounting arm (18) and the second mounting arm (20).

15. A method for positioning a probe holder (16) using the probe holder positioning device (14) according to any one of claims 1 to 13, the method comprising at least the following steps:
connecting the probe holder positioning device (14) to a table (12) via the first mounting arm (18) and the second mounting arm (20);
connecting the probe holder (16) to the probe holder positioning device (14);
connecting a diagnostic or therapeutical probe (17) to the probe holder (16);
providing a desired position of the probe holder positioning device (14) relative to the table (12);
moving the probe holder positioning device (14) to the desired position; and
moving the locking device (70) to the locking position for locking the probe holder positioning device (14) and the probe holder (16) in the desired position.
